# EUROPÄISCHE PATENTSCHRIFT

(11) **EP 0 908 451 B1**
(45) Veröffentlichungstag und Bekanntmachung des Hinweises auf die Patenterteilung: **10.12.2003**
(21) Anmeldenummer: 98118720.6
(22) Anmeldetag: 02.10.1998
(51) Int. Cl.: C07D 223/10, C07D 207/38, C07D 211/40, C07D 225/02, C08L 61/32, D21H 17/51

(54) **Emissionsarme Modifizierungsmittel für Melamin-Tränkharze und diese enthaltende Harzmischungen**
Low-emission modifier for melamine impregnation resins and resin compositions containing them
Modificateur à faible émission pour résines à base de mélamine et compositions de résine les contenant

(30) Priorität: 10.10.1997 DE 19744942
(43) Veröffentlichungstag der Anmeldung: 14.04.1999
(73) Patentinhaber: Surface Specialties Germany GmbH & Co. KG, 65203 Wiesbaden (DE)
(72) Erfinder: Dörries, Peter, 60388 Frankfurt (DE); Wonner, Johann Dr., 63069 Offenbach (DE)
(74) Vertreter: Deckers, Hellmuth, Dr.

(56) Entgegenhaltungen:
- EP-A- 0 275 551
- EP-A- 0 747 531
- DE-A- 2 114 295
- DE-A- 2 328 431
- DE-A- 2 616 374
- DE-A- 2 637 424
- DE-A- 2 755 588
- DE-A- 2 755 589
- DE-A- 3 506 473
- FR-A- 1 546 430
- FR-A- 2 481 299
- US-A- 4 760 152

## Beschreibung

Die vorliegende Erfindung betrifft Lactamderivate und insbesondere deren Mischungen, die durch Umsetzungen von Lactamen der Formel I mit Formaldehyd und Diolen bzw. Diol-mono-äthem der Formel II und/oder der Formel III

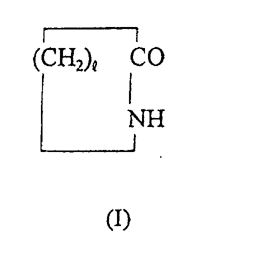

H(-O-CH₂-CR¹H)ₘ-OR² (II)

HO(-CH₂)ₙ-OR³ (III)

herstellbar sind sowie ihre Verwendung als Modifizierungsmittel für Melaminharze. Dabei bedeutet ℓ eine ganze Zahl von 3 bis 12, bevorzugt 3, 4, 5 und 11, m eine ganze Zahl von 1 bis 20, bevorzugt 2 bis 4 und n eine ganze Zahl von 2 bis 8, bevorzugt 2 bis 4; R¹, R² und R³ bedeuten jeweils unabhängig voneinander Wasserstoff oder einen linearen oder verzweigten Alkylrest mit 1 bis 6, bevorzugt 1 bis 4, und besonders bevorzugt 1 bis 2 Kohlenstoffatomen.

Die Herstellung von verschiedenen Umsetzungsprodukten von ε-Caprolactam, Formaldehyd und Hydroxylgruppen-haltigen Verbindungen ist bekannt: z. B. das N[(Isopentyloxy)methyl]-caprolactam aus V. A. Chauzov et al., Journal of General Chemistry of the USSR, **59** (1989) S. 425; das N[(2,6-di-tert.-butylphenoxy)methyl]caprolactam aus der DE-A 26 16 374. Die Herstellung von N,N-verknüpften Bis-lactamverbindungen wird in der DE-A 35 06 473, die Herstellung von N-Methylolcaprolactam in der DE-A 37 00 451 beschrieben. In der EP-A 0 747 531 ist eine Harzmischung für die Imprägnierung von Papier beschrieben, enthaltend ein mit Poly-(C₂ bis C₄-alkylenoxid)-Gruppen N-substituiertes C₂- bis C₂₀-Lactam. In der DE-A 27 55 588 sind N-substituierte ε-Caprolactam-Verbindungen beschrieben, erhältlich aus ε-Caprolactam, Formaldehyd und Formamid. Aus der DE-A 27 55 589 schließlich sind mit diesen Verbindungen modifizierte Aminoplaste bekannt.

Melaminharze aus Melamin und Formaldehyd sind allgemein bekannt und werden z. B. im "Kunststoff-Handbuch", 2. Auflage, 1988, Bd. **10**, S. 41 bis 49 beschrieben. Melamin-Formaldehyd-Vorkondensate, deren Methylolgruppen unveräthert oder mit Alkoholen wie Methanol teilweise veräthert sind, werden in der Regel im wäßrigen Medium hergestellt und auch in wäßriger Form in den Handel gebracht. Sie dienen vorzugsweise zum Tränken von Papierbahnen, insbesondere von dekorativen Papierbahnen, die dann zur Herstellung von Laminaten, dekorativ beschichteten Spanplatten oder Schichtpreßstoffen eingesetzt werden. Dazu werden die Papierbahnen in den gegebenenfalls mit einem Härter versetzten wäßrigen Tränkharzlösungen bis zu einem bestimmten Harzanteil imprägniert und bei Temperaturen von 120 bis 200 °C auf einen bestimmten Restfeuchtegehalt getrocknet. Die so behandelten Gewebe- oder Papierbahnen werden auf Holzwerkstoffe oder einen Stapel beharzter Papiere aufgepreßt, wobei Preßdrücke von 0,8 bis 12 MPa (8 bis 120 bar) und Temperaturen von 100 bis 180 °C angewandt werden.

Auf diese Weise werden dekorative Schichtstoffe und beschichtete Holzwerkstoffe erhalten, die vornehmlich im Innenausbau zur Herstellung von Möbeln oder als Fußbodenbelag eingesetzt werden, um nur einige Anwendungsgebiete zu nennen.

Melaminharze werden durch Kondensation von Formaldehyd mit Melamin hergestellt, wobei die Kondensation nur soweit durchgeführt wird, daß die Reaktionsprodukte noch löslich und schmelzbar bleiben. Wenn dieser Zustand erreicht ist, wird die Kondensation durch Abkühlen und Einstellen eines schwach alkalischen pH-Wertes abgebrochen. Man erhält so nicht voll auskondensierte Produkte, die auch als Melaminharzvorkondensate bezeichnet werden und die in Form ihrer wäßrigen Lösungen z. B. als Tränkharze verwendet werden. Es ist im Rahmen der Erfindung auch möglich, bis zu 20 % des Melamins durch einen oder mehrere andere Aminoplastbildner, wie beispielsweise Guanamine (z. B. Acetoguanamin, Benzoguanamin und Caprinoguanamin), Dicyandiamid, Harnstoff und Thioharnstoff sowie cyclische Harnstoffe (z. B. Äthylen- und Propylenharnstoff) zu ersetzen.

Bei der Verpressung der mit Tränkharz imprägnierten und getrockneten Gewebe- oder Papierbahnen zu Laminaten erfolgt eine Aushärtung infolge einer durchgehenden Vernetzung des Kondensates. Es ist vor allem bei der Beschichtung von Holzwerkstoffen wichtig, daß eine Elastifizierung der eingesetzten Melaminharze mit Modifizierungsmitteln vorgenommen wird, um eine nachträgliche Rißbildung der beschichteten Oberfläche zu vermeiden. Das Tränkharz kann zum Teil mit niederen Alkoholen veräthert oder mit Modifizierungsmitteln wie mehrwertigen Alkoholen, Carbonsäureamiden, Glykolen, Sulfonamiden und Zucker modifiziert und auch mit sauer reagierenden anorganischen oder organischen Salzen katalysiert sein.

Als Modifizierungsmittel werden nach dem Stand der Technik häufig Diäthylenglykol und ε-Caprolactam eingesetzt. Diese verleihen den ausgehärteten Filmen die notwendige Elastizität bei gleichzeitig gegebenen guten Oberflächeneigenschaften wie geringe Anschmutzbarkeit, geringe Empfindlichkeit gegen Wasserdampf und kochendes Wasser, geringe Vergilbungsneigung, hohe Kratzbeständigkeit, geringe Neigung zur Rißbildung und guter "Post Forming"-Eigenschaften.

Nachteilig an diesen Modifizierungsmitteln ist deren verhältnismäßig hohe Flüchtigkeit. Beim Trocknen der mit derart modifizierten Harzen imprägnierten Papiere wird ein Teil der enthaltenen Modifizierungsmittel an die Luft abgegeben. Dieses Problem tritt insbesondere dann auf, wenn aufgrund höherer Maschinengeschwindigkeiten die Temperatur der Trockner erhöht wird. Übliche Trocknungstemperaturen liegen nach dem Stand der Technik bei 140 bis 200 °C. Um diese Emissionen zu vermeiden, sind daher mit Harzen nach dem Stand der Technik im allgemeinen erhebliche Investitionen in Filter- bzw. Nachverbrennungsanlagen notwendig.

Dieses Problem gilt besonders für ε-Caprolactam, für das der MAK-Wert auf 5 mg/m³ festgelegt wurde.

Aufgabe der vorliegenden Erfindung war es deshalb, Modifizierungsmittel für Tränkharze bzw. damit modifizierte Harze bereitzustellen, deren Verwendung bei der Beschichtung von Holzwerkstoffen dazu führt, daß möglichst geringe Mengen an organischen Verbindungen emittiert werden. Außerdem sollen die mit diesen Harzen hergestellten Beschichtungen elastisch sein und nicht zur Rißbildung neigen.

Es wurde nun gefunden, daß man die Nachteile des Einsatzes von Modifizierungsmitteln nach dem Stand der Technik vermeiden kann, wenn man als Modifizierungsmittel Umsetzungsprodukte einsetzt von Lactamen der Formel I, 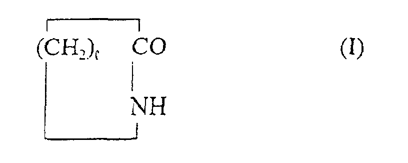 worin ℓ für 3 bis 12, bevorzugt 3, 4, 5 oder 11, besonders bevorzugt für 5 (Caprolactam) und 11 (Laurinlactam) steht,
mit Formaldehyd und Diolen bzw. Diol-mono-äthern der Formel II,

H(OCH₂-CR¹H)ₘ-OR² (II)

worin R¹ und R² Wasserstoff oder einen linearen oder verzweigten Alkylrest mit 1 bis 6, bevorzugt 1 bis 4, und besonders bevorzugt 1 bis 2 Kohlenstoffatomen, bedeutet und m für 1 bis 20, bevorzugt für 2 bis 4, steht, oder Diolen bzw. Diol-mono-äthern der Formel III,

HO-(CH₂)ₙ-OR³ (III)

worin R³ Wasserstoff oder einen linearen oder verzweigten Alkylrest mit 1 bis 6, bevorzugt 1 bis 4, und besonders bevorzugt 1 bis 2 Kohlenstoffatomen, bedeutet und n für 2 bis 8, vorzugsweise 2 bis 4 steht. Es ist erfindungsgcmäß auch möglich, Mischungen verschiedener Lactame einzusetzen, ebenso können auch Mischungen der Diole oder Diol-Monoäther der Formeln II bzw. III eingesetzt werden.

Zur Herstellung der Umsetzungsprodukte wird das Stoffmengenverhältnis der Edukte (Ausgangsstoffe) so gewählt, daß pro 1 mol des Lactams der Formel I 1 bis 4 mol Formaldehyd und 0,5 bis 6 mol des Diols bzw. des Diol-mono-äthers der Formel II und/oder 0,5 bis 6 mol des Diols bzw. Diol-mono-äthers der Formel III miteinander zur Reaktion gebracht werden. Wird das Diol bzw. der Diol-mono-äther der Formel II und/oder der Formel III im Überschuß zur Umsetzung mit dem Lactam der Formel I eingesetzt, so ist es zusätzlich als Lösungsmittel wirksam.

Die Reaktion zwischen dem Lactam der Formel I, Formaldehyd und dem Diol bzw. Diol-mono-äther der Formel II und/oder der Formel III erfolgt bei Temperaturen zwischen 50 und 200 °C, vorzugsweise zwischen 80 und 140 °C, in Gegenwart bekannter wasserabspaltender Katalysatoren, vorzugsweise eines sauren Katalysators. Als saure Katalysatoren eignen sich beispielsweise anorganische oder starke organische Säuren, wie z. B. Amidosulfonsäure, Phosphorsäure, Salzsäure, Salpetersäure, Schwefelsäure, Ameisensäure, Oxalsäure, Benzolsulfonsäure oder Toluolsulfonsäure sowie sauer reagierende Salze wie z. B. Alkalihydrogensulfate.

Es ist auch möglich, die Reaktion zwischen dem Lactam der Formel I, Formaldehyd und dem Diol bzw. Diol-mono-äther der Formel II und/oder der Formel III in zwei Stufen durchzuführen, wobei in der ersten Stufe zunächst in Gegenwart von alkalisch reagierenden anorganischen oder organischen Verbindungen wie z. B. Alkalihydroxyden, Alkali- oder Erdalkalicarbonaten, sowie Aminen, bei Temperaturen zwischen 50 und 200 °C, vorzugsweise zwischen 80 und 120 °C, das Lactam I und Formaldehyd miteinander umgesetzt werden in dem oben genannten Stoffmengenverhältnis. Dabei wird das Lactam der Formel I methyloliert. Die weitere Kondensation erfolgt dann in der zweiten Stufe in Gegenwart bekannter wasserabspaltender Katalysatoren, vorzugsweise eines sauren Katalysators. Als saurer Katalysator eignen sich die oben genannten anorganischen und starken organischen Säuren.

Zur Vervollständigung der Umsetzung kann während der Umsetzung oder danach das Reaktionswasser abdestilliert werden, wobei auch unter vermindertem Druck gearbeitet werden kann. Dabei kann die Kondensationsreaktion auch in Gegenwart von nicht mit Wasser mischbaren Lösungsmitteln, vorzugsweise inerten aromatischen Kohlenwasserstoffen, durchgeführt werden, die mit Wasser ein Azeotrop bilden können. Der Einsatz inerter aromatischer Kohlenwasserstoffe als Lösungsmittel für die Umsetzung ist besonders dann von Vorteil, wenn Formaldehyd in Form von wäßrige Formaldehyd-Lösungen eingesetzt wird, da dann die Entfernung des eingeschleppten Wassers durch azeotrope Destillation begünstigt wird. In diesem Fall kann das Wasser durch azeotrope Destillation weitgehend oder vollständig aus der Produktmischung entfernt werden, wobei das Lösungsmittel in bekannter Weise nach der Phasentrennung vom Wasser zurückgeführt werden kann.

Der Formaldehyd kann in Form einer wäßrigen Lösung, vorzugsweise einer über 30 %igen Lösung, oder in Form einer Lösung von Formaldehydgas in dem bei der Umsetzung eingesetzten Diol oder Diol-mono-äther der Formel II oder III oder auch in einem niedrigsiedenden Alkohol eingesetzt werden. Vorzugsweise wird der Formaldehyd jedoch in der Form von Paraformaldehyd der Reaktion zugeführt. Werden wäßrige Lösungen von Formaldehyd oder Lösungen in niedrigsiedenden Alkoholen eingesetzt, so ist dafür zu sorgen, daß das mit der Formaldehydlösung eingebrachte Wasser bzw. der niedere Alkohol aus dem Reaktionsgemisch abdestillieren kann, was insbesondere in Gegenwart von Wasser durch Zusatz eines organischen Lösungsmittels, das mit dem Wasser ein azeotropes Gemisch bildet, begünstigt wird. Wird mit Paraformaldehyd als Formaldehydquelle gearbeitet, so kann auf eine destillative Entfernung des Reaktionswassers verzichtet werden.

Die nach dem erfindungsgemäßen Verfahren erhältlichen Umsetzungsprodukte sind Mischungen verschiedener Lactamderivate. Bei der Umsetzung reagiert das Lactam der Formel I mit dem Formaldehyd unter Ausbildung von N-Methylol-Lactamen der Formel IV, 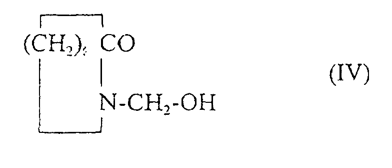 N,N'-Methylenbislactamen der Formel V 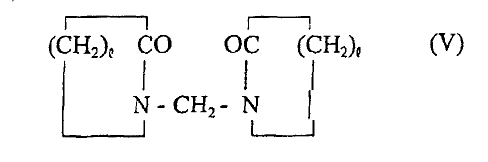 sowie mit dem Formaldehyd und dem eingesetzen Diol bzw. Diol-mono-äther der Formel II und/oder dem Diol der Formel III zu neuartigen Kondensationsprodukten der Formel VI, 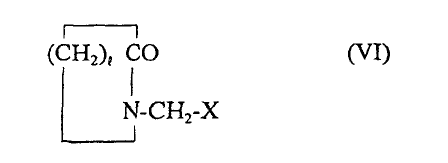 worin X -(OCH₂-CR¹H)ₘ-OR²
bedeutet und R¹, R², ℓ und m die oben angegebene Bedeutung haben,
oder
worin X -O-(CH₂)ₙ-OR³
bedeutet, und R³, ℓ und n die oben angegebene Bedeutung haben.

Die Lactam-Derivate der Formel VI, die durch das erfindungsgemäße Verfahren erhältlich sind, sind bisher noch nicht beschrieben worden; sie sind ebenfalls Gegenstand der vorliegenden Erfindung.

Die nach dem erfindungsgemäßen Verfahren erhältlichen Gemische enthalten Massenanteile von 5 bis 50 %, bevorzugt 10 bis 45 %, an N-Alkoxymethyllactamen der Formel VI, 30 bis 80 %, bevorzugt 35 bis 75 %, an N,N'-Methylen-bis-lactamen der Formel V und 0 bis 5 %, bevorzugt bis zu 2 %, an nicht umgesetzten Lactamen der Formel I.

Als Lactame können beispielsweise 2-Pyrrolidon (γ-Butyrolactam), 2-Piperidon (δ-Valerolactam), ε-Caprolactam und Laurinlactam jeweils einzeln oder in Mischung verwendet werden. Besonders bevorzugt ist ε-Caprolactam.

Als Diole können Äthylenglykol, 1,2- und 1,3-Propylenglykol, 1,4-Butandiol sowie die oligomeren Oxyäthylen- und Oxypropylenglykole mit Polymerisationsgraden von 2 bis 20, bevorzugt 2 bis 4, eingesetzt werden, wobei auch gemischte Oligomere mit Oxyäthylen- und Oxypropylen-Bausteinen verwendet werden können. Bevorzugt werden Oligo-Oxyäthylen- und Oxypropylenglykole mit Polymerisationsgraden von 2 bis 4, besonders Diäthylenglykol.

Die erfindungsgemäß einsetzbaren Diol-Monoäther sind einseitig verätherte Diole, die aus den oben aufgeführten ausgewählt werden. Dabei hat die Alkylgruppe 1 bis 6, bevorzugt 1 bis 4, und besonders bevorzugt 1 bis 2 Kohlenstoffatome; speziell sind die Monomethyl- und Monoäthyläther von Äthylenglykol und Diäthylenglykol zu nennen.

Die genannten Diole und Diol-Monoäther können jeweils einzeln oder in Mischung verwendet werden.

Ganz besonders bevorzugt sind Modifizierungsmittel, die durch Umsetzung von ε-Caprolactam, Formaldehyd und Diäthylenglykol erhalten werden können.

Die rohen, vorzugsweise tel quel verwendeten, erfindungsgemäß hergestellten Mischungen können als Modifizierungsmittel für Melaminharze (Tränkharze) in jeder Phase der Melaminharz-Herstellung zugesetzt werden.

Es ist jedoch auch möglich, die N-Alkoxymethyl-lactame VI z.B. durch fraktionierte Fällung abzutrennen und allein als Modifizierungsmittel einzusetzen.

Besonders wertvolle Melamin-Tränkharz-Zubereitungen erhält man, wenn die erfindungsgemäßen Modifizierungsmittel Melaminharzen mit einem Stoffmengen-Verhältnis von Melamin zu Formaldehyd von 1 mol : 1,4 mol bis 1 mol : 1,8 mol zusetzt. Die erfindungsgemäßen Lactamderivate bzw. die Produktmischungen werden den zu modifizierenden Harzen in einer solchen Menge zugesetzt, daß das Verhältnis *m*_{L}/*m*_{MH} der Masse *m*_{L} des Lactamderivats (bzw. der Produktmischung) zur Masse *m*_{MH} des Feststoffes des Melaminharzes 1 bis 20 %, vorzugsweise 2 bis 10 % beträgt, wobei der Zusatz vor, während oder nach der Kondensation des Harzes erfolgen kann. Neben den erfindungsgemäßen Lactamderivaten können auch zusätzlich Modifizierungsmittel nach dem Stand der Technik, wie z. B. Butandiol, ε-Caprolactam, Diäthylenglykol, Phenoxyäthanol oder Zucker in Massenanteilen von zusammen 2 bis 20 %, bevorzugt 5 bis 15 %, zugesetzt werden, bezogen auf die Masse des Feststoffes des modifizierten Harzes.

Die so erhaltenen, erfindungsgemäß modifizierten Harze (Melamin-Tränkharz-Zubereitungen) haben eine sehr gleichmäßige Qualität, gute Lagerstabilität und eignen sich hervorragend zur Imprägnierung von Dekorpapier, welches in üblicher Weise auf allen gängigen Maschinentypen sowohl nach dem Kurztaktverfahren zur Herstellung von vergüteten Spanplatten als auch nach dem C.P.L.-Verfahren ("continuously pressed laminates") oder H.P.L.-Verfahren ("high pressure laminates") zur Herstellung von Laminaten verarbeitet werden kann. Die erhaltenen Oberflächen sind chemisch und mechanisch sehr resistent, weisen eine hohe Elastizität auf, sind frei von Rissen und zeigen einen hohen und gleichmäßigen Glanz.

Insbesondere treten bei der Verwendung der erfindungsgemäßen Melamin-Tränkharz-Zubereitungen verglichen mit Harzen nach dem Stand der Technik deutlich geringere Emissionen beim Trocknen der beharzten Papiere auf.

Die Herstellung von dekorativ beschichteten Holzwerkstoffplatten unter Verwendung der erfindungsgemäßen Melamin-Tränkharz-Zubereitungen erfolgt so, daß die Papier- bzw. Gewebebahn mit einem erfindungsgemäß modifizierten Melaminharz getränkt und in an sich bekannter Weise weiterverarbeitet wird (Vergl. z. B. Ullmanns Enzyklopädie der techn. Chemie, 4. Auflage, Band 7 (1974), S. 417 f.).

In den nachfolgenden Beispielen bedeuten ebenso wie im vorhergehenden Text alle Angaben mit der Einheit "%" Massenanteile, soweit nicht anders angegeben. "Teile" sind stets Massenteile. Konzentrationsangaben in "%" sind Massenanteile des gelösten Stoffes in der Lösung, soweit nicht anders angegeben.

Die folgenden Meßmethoden werden zur Charakterisierung eingesetzt:
- nichtflüchtige Anteile:: Eine Probe mit der Masse *m*_{P} = 2 g wird in einem Wägeschälchen aus Glas (G) oder Aluminium (Al) eine Stunde lang in einem Trockenschrank bei 120 °C getrocknet. Die Masse des Trockenrückstandes ist *m*_{T}, bei Verwendung von Glasschälchen *m*_{T,G}, bei Verwendung von Aluminiumschälchen *m*_{T,Al}. Angabe des Massenanteiles des Trocken-Rückstandes *w*_{T,G} = *m*_{T,G} / *m*_{P} ( = nfA(Glas)) bzw. *w*_{T,Al} = *m*_{T,Al} / *m*_{P} (= nfA(Alu)).
- Wasserverdünnbarkeit:: zu 1 Teil Harz mit der Masse *m*_{H} bzw. dem Volumen *V*_{H} wird voll entsalztes (VE-) Wasser bei 20 °C langsam zugegeben, bis sich eine deutliche Trübung einstellt. Die dazu erforderliche Wassermenge sind t Teile mit der Masse *m*_{W} bzw. dem Volumen *V*_{W}. Angabe des Volumen-Verhältnisses ψ_{WH} = *V*_{W} / *V*_{H} (t Volumenteile Wasser, 1 Volumenteil Harz) in cm³/cm³ oder des Massenverhältnisses ζ_{WH} = *m*_{W} / *m*_{H} (t Massenteile Wasser, 1 Massenteil Harz) in g/g.
- Auslaufzeit:: Auslaufzeit einer Flüssigkeit aus einem Auslaufbecher gemäß DIN 53 211 mit einer Auslauföffnung des Durchmessers 4 mm

### Beispiele

### A. Herstellung der Modifizierungsmittel

### Beispiel 1

In einem 4 l Dreihalskolben mit KPG-Rührer, Rückflußkühler und Innenthermometer wurden 1486 g (14,0 mol) Diäthylenglykol, 1584 g (14,0 mol) ε-Caprolactam, 924 g Paraformaldehyd 91 %ig (Granuform®, Degussa, 28,0 mol) und 13,3 g p-Toluolsulfonsäure·H₂O (0,070 mol) vorgelegt und unter Rühren in 90 min auf 100 °C erhitzt. Die Reaktionsmischung wurde 30 min bei 100 °C gerührt. Dabei ging der Ansatz in Lösung. Die Kondensation wurde durch Abkühlen auf 30 °C und Einstellen des pH-Wertes mit ca. 11 g 50 %iger Natronlauge auf pH 8 abgebrochen. Das fertige Modifizierungsmittel ist mit Wasser unbegrenzt mischbar und hat folgende Kennzahlen:

| | |
|---|---|
| nicht-flüchtiger Anteil (Alu) | *w*_{T,Al} = 77 % |
| pH (20 °C) | 8,3 |
| Konzentration an freiem Formaldehyd (DIN 16 746 A) | 7,6% |
| Brookfield-Viskosität (23 °C, LV 2, 60 min⁻¹) | 158 mPa·s |

Nach quantitativem ¹³C-NMR (H₂O + 10% DMSO) liegen folgende Spezies in der Mischung vor:

| | |
|---|---|
| ε-Caprolactam | 24,1; 30,1; 31,3; 37,0; 43,7; 182,9 ppm. |
| N,N'-Methylenbiscaprolactam | 24,2; 28,9; 30,5; 37,6; 49,7; 58,8; 180,9 ppm. |
| N-(7-Hydroxy-2,5-dioxaheptyl)caprolactam | 24,3; 29,3; 30,6; 37,9; 50,1; 62,0; 68,6; 71,1; 73,2; 78,3; 181,1 ppm. |
| Diäthylenglykol | 61,9; 73,3 ppm. |

Vom zur Umsetzung eingesetzten ε-Caprolactam lagen 1% als freies ε-Caprolactam, 42 % als N,N'-Methylenbiscaprolactam und 24 % als N-(7-Hydroxy-2,5-dioxaheptyl)caprolactam in der Mischung vor. 33 % konnten keiner definierten Verbindung zugeordnet werden.

Von dem zur Umsetzung eingesetzten Diäthylenglykol lagen 24 % als N-(7-Hy-droxy-2,5-dioxaheptyl)caprolactam und 49 % als freies Diäthylenglykol vor. 27 % konnten keiner definierten Verbindung zugeordnet werden.

### Beispiel 2

In einem 4 l Dreihalskolben mit KPG-Rührer, Rückflußkühler und Innenthermometer wurden 2122 g (20 mol) Diäthylenglykol, 2263 g (20 mol) ε-Caprolactam, 1320 g Paraformaldehyd 91%ig (Granuform®, Degussa, 40 mol) und 171 g p-Toluolsulfonsäure·H₂O (0,90 mol) vorgelegt und unter Rühren in 30 min auf 100 °C erhitzt. Die Reaktionsmischung wurde 10 min bei 100 °C gerührt. Dabei ging der Ansatz in Lösung. Die Kondensation wurde durch Abkühlen auf 30 °C und Einstellen des pH-Wertes mit ca. 105 g 50 %iger Natronlauge auf pH 8 abgebrochen.

Vom zur Umsetzung eingesetzten ε-Caprolactam lagen nach ¹³C-NMR 1 % als freies ε-Caprolactam, 50 % als N,N'-Methylenbiscaprolactam und 23 % als N-(7-Hydroxy-2,5-dioxaheptyl)caprolactam in der Mischung vor. 26 % konnten keiner definierten Verbindung zugeordnet werden.

### Beispiel 3

In einem 0,5 l Dreihalskolben mit KPG-Rührer, Rückflußkühler und Innenthermometer wurden 212 g (2,0 mol) Diäthylenglykol, 226 g (2,0 mol) ε-Caprolactam, 66 g Paraformaldehyd 91 %ig (Granuform®, Degussa, 2,0 mol) und 0,80 g (10 mmol) 50 %ige Natronlauge vorgelegt und 30 min bei 100 °C gerührt. Dann wurden 19 g (0,10 mol) p-Toluolsulfonsäure·H₂O zugesetzt und noch 10 min bei 100 °C gerührt. Die Kondensation wurde durch Abkühlen auf 30 °C und Einstellen des pH-Wertes mit ca. 9 g (0,11 mol) 50 %iger Natronlauge auf pH 8 abgebrochen. Von dem zur Umsetzung eingesetzten ε-Caprolactam lagen nach ¹³C-NMR 5 % als freies ε-Caprolactam, 65 % als N,N'-Methylenbiscaprolactam und 22 % als N-(7-Hydroxy-2,5-dioxaheptyl)caprolactam in der Mischung vor; 8 % konnten keiner definierten Verbindung zugeordnet werden. 47 % des eingesetzten Diäthylenglykols lagen frei vor.

### Beispiel 4

Durchführung wie Beispiel 3. Nach Zugabe der p-Toluolsulfonsäure wurde in einem Zeitraum von 2 Stunden bis auf eine Innentemperatur von 140 °C erhitzt und dabei insgesamt 23 g Wasser abdestilliert. Die Kondensation wurde durch Abkühlen auf 30 °C und Einstellen des pH-Wertes mit ca. 9 g (0,11 mol) 50 %iger Natronlauge auf pH 9 abgebrochen.
Von dem zur Umsetzung eingesetzten ∈-Caprolactam lagen nach ¹³C-NMR 6 % als freies ε-Caprolactam, 70 % als N,N'-Methylenbiscaprolactam und 24 % als N-(7-Hydroxy-2,5-dioxaheptyl)caprolactam in der Mischung vor. 52 % des eingesetzten Diäthylcnglykols lagen frei vor.

### Beispiel 5

Mit 1,4-Butandiol bzw. Polyäthylenglykol 200 (PEG 200) wurden analog zu Beispiel 1 Modifizierungsmittel hergestellt. Die Ansätze wurden 30 Minuten bei 100 °C gerührt, abgekühlt und mit einer zur Säuremenge äquivalenten Menge Natronlauge neutralisiert. Die eingesetzten Mengen und Kennzahlen der erhaltenen Modifizierungsmittel sind untenstehender Tabelle zu entnehmen:

| Beispiel | Zusammensetzung | | Kennzahlen | | |
|---|---|---|---|---|---|
| | | | nfA(Glas) | pH(1:1) | Auslaufzeit |
| 5 A | 339,5 g (3,0 mol) | ε-Caprolactam | 89 % | 8,3 | 29 s |
| | 270,4 g (3,0 mol) | 1,4-Butandiol | | | |
| | 99,0 g (3,0 mol) | Paraformaldehyd, 91%ig | | | |
| | 2,85 g (0,015 mol) | p-Toluolsulfonsäure·H₂O | | | |
| 5 B | 339,5 g (3,0 mol) | ε-Caprolactam | 83% | 6,7 | 28 s |
| | 270,4 g (3,0 mol) | 1,4-Butandiol | | | |
| | 198,0 g (6,0 mol) | Paraformaldehyd, 91%ig | | | |
| | 2.85 g (0,015 mol) | p-Toluolsulfonsäure·H₂O | | | |
| 5 C | 282,9 g (2,5 mol) | ε-Caprolactam | 92 % | 5,8 | 30 s |
| | 500,0 g (2,5 mol) | Polyäthylenglykol 200 | | | |
| | 82,5 g (2,5 mol) | Paraformaldehyd, 91%ig | | | |
| | 2,38 g (0,013 mol) | p-Toluolsulfonsäure·H₂O | | | |
| 5 D | 282,9 g (2,5 mol) | ε-Caprolactam | 90 % | 5,4 | 37 s |
| | 500,0 g (2,5 mol) | Polyäthylenglykol 200 | | | |
| | 165,0 g (5,0 mol) | Paraformaldehyd, 91%ig | | | |
| | 2,38 g (0.013 mol) | p-Toluolsulfonsäure·H₂O | | | |
| pH (1:1): pH-Wert der mit VE-Wasser im Massenverhältnis 1:1 verdünnten Probe. | | | | | |

### B. Herstellung der Tränkharze

### Beispiel 6

In einem 1 l Dreihalskolben mit KPG-Rührer, Rückflußkühler und Innenthermometer wurden 219,3 g voll entsalztes Wasser, 88 g Modifizierungsmittel aus Beispiel 1 und 395 g (5,1 mol) 39 %iger Formaldehyd vorgelegt. Dann wurden 0,4 g (5 mmol) 50 %ige Natronlauge und anschließend 454 g (3,6 mol) Melamin zugesetzt. Der pH-Wert (23 °C) der Reaktionsmischung betrug 9,9.
Der Ansatz wurde auf Rückflußtemperatur (ca. 103 °C) angeheizt und 10 min bei Rückfluß gerührt, dann auf 85 °C abgekühlt und bis zu einer Wasserverdünnbarkeit ψ_{WH}= 2 (1 Vol. Teil Harz zu 2 Vol. Teile VE-Wasser) kondensiert. Die Kondensation wurde durch Abkühlen auf 30 °C abgebrochen.
Das erhaltene Harz hatte einen Massenanteil *w*_{t,Al} von nichtflüchtigen Anteilen von 59 %.

### Beispiel 7

In einem 50 l Kessel mit Rührer, Rückflußkühler und Innenthermometer wurden 9,3 kg VE-Wasser, 4,0 kg Modifizierungsmittel aus Beispiel 2 und 17,1 kg (223 mol) 39 %iger Formaldehyd vorgelegt. Dann wurden 17,4 g (0,22 mol) 50 %ige Natronlauge und anschließend 19,6 kg (156 mol) Melamin zugesetzt. Der pH-Wert (23 °C) der Reaktionsmischung betrug 9,6. Der Ansatz wurde auf Rückflußtemperatur (ca. 103 °C) angeheizt und 10 min bei Rückfluß gerührt, dann auf 85 °C abgekühlt und bis zu einer Wasserverdünnbarkeit von 1 Vol. Teil Harz zu 1,9 Vol. Teile VE-Wasser kondensiert. Die Kondensation wurde durch Abkühlen auf 30 °C abgebrochen. Das erhaltene Harz hatte folgende Kennzahlen:

| | |
|---|---|
| nfA(Glas): | *w*_{T,G} = 60,6 % |
| Dichte (20 °C): | ρ = 1247 kg/m³ |
| pH | 9,7 |
| Auslaufzeit | 18,0 s |
| Wasserverdünnbarkeit | ζ_{WH} = 1,6 g/g |
| Haltbarkeit | ca. 4 Wochen |

### Beispiel 8

Mit den Modifizierungsmitteln aus Beispiel 5 wurden analog zu Beispiel 6 Tränkharze hergestellt. Die Zusammensetzung der Ansätze sowie die Kennzahlen der Harze sind untenstehender Tabelle zu entnehmen:

| Beispiel | Zusammensetzung | | Kennzahlen | | | |
|---|---|---|---|---|---|---|
| | | | nfA (Alu) | ζ_{WH} | pH | Auslaufzeit |
| 8 A | 184,0g | VE-Wasser | 59 % | 1,7 g/g | 9,2 | 15 s |
| | 72,4 g | Mod.mittel aus Beispiel 5 A | | | | |
| | 2,24 ml | 2 N Natronlauge | | | | |
| | 369,8 g | 39,1%iger wäßriger Formaldehyd | | | | |
| | 403,5 g | Melamin | | | | |
| 8 B | 197,3 g | VE-Wasser | 59 % | 1,4 g/g | 9,2 | 17 s |
| | 82,5 g | Mod.mittel aus Beispiel 5 B | | | | |
| | 2,24 ml | 2 N Natronlauge | | | | |
| | 346,4 g | 39,1%iger wäßriger Formaldehyd | | | | |
| | 403,5 g | Melamin | | | | |
| 8 C | 179,4g | VE-Wasser | 60 % | 1,6 g/g | 9,1 | 16 s |
| | 68,7 g | Mod.mittel aus Beispiel 5 C | | | | |
| | 2,24 ml | 2 N Natronlauge | | | | |
| | 378,0 g | 39,1%iger wäßriger Formaldehyd | | | | |
| | 403,5 g | Melamin | | | | |
| 8 D | 188,1 g | VE-Wasser | 60 % | 1,6 g/g | 9,2 | 17 s |
| | 75,3 g | Mod.mittel aus Beispiel 5 D | | | | |
| | 2,24 ml | 2 N Natronlauge | | | | |
| | 362,9 g | 39,1%iger wäßriger Formaldehyd | | | | |
| | 403,5 g | Melamin | | | | |

### Vergleichsbeispiel 1

In einem 50 l Kessel mit Rührer, Rückflußkühler und Innenthermometer wurden 8,3 kg VE-Wasser, 1,5 kg ε-Caprolactam, 1,4 kg Diäthylenglykol und 19,1 kg (249 mol) 39 %iger Formaldehyd vorgelegt. Dann wurden 17,4 g (0,22 mol) 50 %ige Natronlauge und anschließend 19,6 kg (156 mol) Melamin zugesetzt. Der pH-Wert (23 °C) der Reaktionsmischung betrug 9,7. Der Ansatz wurde auf Rückflußtemperatur (ca. 103 °C) angeheizt und 10 min bei Rückfluß gerührt, dann auf 85 °C abgekühlt und bis zu einer Wasserverdünnbarkeit ψ_{WH} = 1,9 (1 Vol. Teil Harz zu 1,9 Vol. Teile VE-Wasser) kondensiert. Die Kondensation wurde durch Abkühlen auf 30 °C abgebrochen.

Das erhaltene Harz hatte folgende Kennzahlen:

| | |
|---|---|
| (nfA (Glas), 2 g, 1 h 120 °C) | *w*_{T,G} = 60,7 % |
| Dichte (20 °C): | ρ = 1245 kg/m³ |
| pH-Wert: | 9,6 |
| Auslaufzeit | 17,5 s |
| Wasserverdünnbarkeit | ζ_{WH} = 1,5 g/g |
| Haltbarkeit | ca. 5 Wochen |

### Vergleichsbeispiel 2

In einem 1 l Dreihalskolben mit KPG-Rührer, Rückflußkühler und Innenthermometer wurden 145 g VE-Wasser und 444 g (5,77 mol) 39 %iger Formaldehyd vorgelegt. Dann wurden 2,5 ml 2 N (5 mmol) Natronlauge und anschließend 454 g (3,6 mol) Melamin zugesetzt. Der pH-Wert (23 °C) der Reaktionsmischung betrug 9,4.

Der Ansatz wurde auf Rückflußtemperatur (ca. 103 °C) angeheizt und 10 min bei Rückfluß gerührt, dann auf 85 °C abgekühlt und bis zu einer Wasserverdünnbarkeit ψ_{WH} = 1,9 (1 Vol. Teil Harz zu 1.9 Vol. Teile VE-Wasser) kondensiert. Die Kondensation wurde durch Abkühlen auf 30 °C abgebrochen. Das erhaltene Harz hatte folgende Kennzahlen:

| | |
|---|---|
| nfA (Alu) | *w*_{T.A} = 58,8% |
| pH-Wert | 9,4 |
| Wasserverdünnbarkeit: | ζ_{WH} = 1,3 g/g |
| Haltbarkeit | 4 Tage |

### Vergleichsbeispiel 3

Zu den Harzen des Beispiels 8 wurden die entsprechenden mit den reinen Modifizierungsmitteln 1,4-Butandiol bzw. Polyäthylenglykol 200 (molare Masse ca. 200 g/mol) und ε-Caprolactam modifizierten Harze zu Vergleichszwecken hergestellt. Die Durchführung erfolgt analog zu Vergleichsbeispiel 1, die Zusammensetzung der Ansätze sowie die Kennzahlen sind untenstehender Tabelle zu entnehmen:

| Vergleichsbeispiel | Zusammensetzung | | Kennzahlen | | | |
|---|---|---|---|---|---|---|
| | | | nfA (Alu) | ζ_{WH} | pH | Auslaufzeit |
| 3 A Vergleich zu Beispiel 8 A und B | 171,1 g | VE-Wasser | 60% | 1,9 g/g | 9,3 | 16 s |
| | 34,5 g | ε-Caprolactam | | | | |
| | 27,4 g | 1,4-Butandiol | | | | |
| | 2,24 ml | 2 N Natronlauge | | | | |
| | 393,2 g | 39,1%iger wäßriger Formaldehyd | | | | |
| | 403.5 g | Melamin | | | | |
| 3 B Vergleich zu Beispiel 8 C und D | 171,1 g | VE-Wasser | 60% | 1,6 g/g | 9,2 | 16 s |
| | 22,4 g | ε-Caprolactam | | | | |
| | 39,6 g | Polyäthylenglykol 200 | | | | |
| | 2,24 ml | 2 N Natronlauge | | | | |
| | 393,2 g | 39,1%iger wäßriger Formaldehyd | | | | |
| | 403,5 g | Melamin | | | | |

### C. Untersuchung des Emissionsverhaltens

### Untersuchung 1

Das Abrauchverhalten von mit den Harzen aus Beispiel 7 und Vergleichsbeispiel 1 imprägnierten Filmen wurde qualitativ beurteilt. Die Bruttozusammensetzung der beiden Harze (Melamin, Formaldehyd, ε-Caprolactam und Diäthylenglykol) ist gleich. Die Harze wurden mit einem sauren Aminsalz (p-Toluolsulfonsäure/Morpholin) auf eine Trübungszeit (T-Zeit) von 5 bis 5½ min bei 100 °C eingestellt. Mit diesen Tränkflotten wurde Dekorpapier imprägniert und im Trockenschrank bei 180 °C getrocknet. Das Abrauchverhalten wurde im Minutenabstand verfolgt. Die Ergebnisse sind in untenstehender Tabelle aufgeführt:

| Harz | T-Zeit 100 °C min | Abrauchverhalten bei 180 °C nach | | | |
|---|---|---|---|---|---|
| | | 1 min | 2 min | 3 min | 4 min |
| Beispiel 7 | 5½ | ohne Befund | ohne Befund | ohne Befund | leichte Rauchentwicklung |
| Vergleichsbeispiel 1 | 5 | ohne Befund | ohne Befund | leichte Rauchentwicklung | deutliche Rauchentwicklung |

### Untersuchung 2

Die Emissionen an ε-Caprolactam (Capro) bzw. Diäthylenglykol (DEG) der Harze aus Beispiel 7, Vergleichsbeispiel 1 und Vergleichsbeispiel 2 wurden mittels GC-Analyse untersucht. Dazu wurden die Harzlösungen bei einer Blocktemperatur von 120 °C bzw. 160 °C direkt injiziert. Die erhaltenen Ergebnisse sind in untenstehenden Tabellen aufgeführt:

GC-Analyse bei 120 °C (flüchtige Anteile bezogen auf Harzlösung):

| Harz | Modifizierung | Capro | DEG | Capro + DEG |
|---|---|---|---|---|
| Beispiel 7 | erfindungsgemäß mit Modifzierungsmittel aus Bsp. 2 | 0,8 % | 0.6 % | 1,4 % |
| Vergl. Beispiel 1 | Caprolactam und Diäthylenglykol | 2,5 % | 2,3 % | 4,8 % |
| Vergl. Beispiel 2 | unmodifiziert | <0,1 % | <0,1 % | <0,1 % |

GC-Analyse bei 160 °C (flüchtige Anteile bezogen auf Harzlösung):

| Harz | Modifizierung | Capro | DEG | Capro + DEG |
|---|---|---|---|---|
| Beispiel 7 | erfindungsgemäß mit Modifizierungsmittel aus Bsp. 2 | 0,4 % | 0.9 % | 1,3 % |
| Vergl. Beispiel 1 | Caprolactam und Diäthylenglykol | 1,9 % | 2,6 % | 4,5 % |
| Vergl. Beispiel 2 | unmodifiziert | <0,1 % | <0,1 % | <0,1 % |

Durch Einsatz des erfindungsgemäßen Tränkharzes aus Beispiel 7, das das erfindungsgemäße Modifizierungsmittel aus Beispiel 2 enthält, lassen sich die Emissionen an ε-Caprolactam und Diäthylenglykol im Vergleich zum Tränkharz aus dem Vergleichsbeispiel 1, welches mit den reinen Modifizierungsmitteln ε-Caprolactam und Diäthylenglykol modifiziert ist, deutlich reduzieren.

### Untersuchung 3

Das Abrauchverhalten von mit den Harzen aus Beispiel 8 und Vergleichsbeispiel 3 imprägnierten Filmen wurde qualitativ beurteilt. Die Bruttozusammensetzung der Harze (Melamin, Formaldehyd, ε-Caprolactam und 1,4-Butandiol) aus Beispiel 8 A, 8 B und Vergleichsbeispiel 3 A sowie die Bruttozusammensetzung der Harze (Melamin, Formaldehyd, ε-Caprolactam und Polyäthylenglykol 200) aus Beispiel 8 C, 8 D und Vergleichsbeispiel 3 B ist jeweils gleich. Die Harze wurden mit einem sauren Aminsalz (p-Toluolsulfonsäure/Morpholin) auf eine Trübungszeit (T-Zeit) von 5 bis 5½ min bei 100 °C eingestellt. Mit diesen Tränkflotten wurde Dekorpapier imprägniert und im Trockenschrank bei 180 °C getrocknet. Das Abrauchverhalten wurde im Minutenabstand verfolgt. Die Ergebnisse sind in untenstehender Tabelle aufgeführt:

| Harz | Abrauchverhalten bei 180 °C nach: Rauchentwicklung | | | |
|---|---|---|---|---|
| | 1 min | 2 min | 3 min | 4 min |
| Beispiel 8 A | ohne Befund | ohne Befund | leicht | deutlich |
| Beispiel 8 B | ohne Befund | ohne Befund | leicht | deutlich |
| Vergleichsbeispiel 3 A | ohne Befund | leicht | deutlich | stark |
| Beispiel 8 C | ohne Befund | ohne Befund | ohne Befund | deutlich |
| Beispiel 8 D | ohne Befund | ohne Befund | ohne Befund | deutlich |
| Vergleichsbeispiel 3 B | ohne Befund | ohne Befund | deutlich | stark |

Durch Einsatz der erfindungsgemäßen Tränkharze aus Beispiel 8 läßt sich die Rauchentwicklung gegenüber dem Stand der Technik (Vergleichsbeispiel 3) deutlich reduzieren.

### D. Anwendungstechnische Ausprüfung

### Untersuchung 4

Die Tränkharze aus Beispiel 7 und Vergleichsbeispiel 1 werden anwendungstechnisch für den Kurztakt-Einsatz geprüft. Dazu wurden die Harzlösungen mit einem sauren Aminsalz (p-Toluolsulfonsäure/Morpholin) auf eine Trübungszeit von ca. 5 min eingestellt. Die mit diesen Harzlösungen imprägnierten Dekorpapiere (PWA, A-60 S, 80 g/m²) wurden im Trockenschrank getrocknet und anschließend auf Spanplatten verpreßt (60 s, 160 °C am Papier, 3 MPa [=30 bar], Preßblech HS-18). Die Ergebnisse der Ausprüfung sind untenstehender Tabelle zu entnehmen:

| Harz | Melaminfilm | | Ausprüfung der beschichteten Spanplatte | | | |
|---|---|---|---|---|---|---|
| | Harzanteil | Restfeuchte | Oberfläche | Kitontest^{‡} 2 h | Überhärtung | Temperung n. DIN 68765, 4.6 |
| Beispiel 7 | 60 % | 6,0 % | ohne Befund | 2-3 | ohne Befund | ohne Befund |
| Vergl. Bsp. 1 | 58 % | 6.1 % | ohne Befund | 2 | ohne Befund | ohne Befund |

| | | | | | | |
|---|---|---|---|---|---|---|
| ‡ **Kitontest**: Prüfung von MF-Harz vergüteten Oberflächen | | | | | | |

- Lösung I:: 1.000 ml H₂O
20 ml H₂SO₄
20 ml 2 %ige wäßrige Lösung ®Lissamine Red B (Hersteller : ICI)

Ein mit der Lösung I gefülltes "Glasauge" (Kalotte) wird auf den Probekörper aufgebracht und bei Raumtemperatur 2 Stunden einwirken gelassen. Hiernach wird die Prüfstelle gründlich mit Wasser gereinigt und mit einer 6-stufigen Farbskala beurteilt.

Die Stufen der Färbung gehen von
Stufe 1: keine Anfärbung = Überhärtung bis
Stufe 6: dunkelviolette Anfärbung = völlige Unterhärtung.
Bei vergüteten Spanplatten sollten Aushärtungen der Stufe 2 bzw. 2-3 (gut, gut - mäßig) angestrebt werden.

Zwischen dem erfindungsgemäßen Harz aus Beispiel 7 und dem Harz des Standes der Technik aus Vergleichsbeispiel 1 bestehen für den Kurztakteinsatz keine anwendungstechnischen Unterschiede.

### Untersuchung 5

Die Tränkharze aus Beispiel 7 und Vergleichsbeispiel 1 wurden anwendungstechnisch für den C.P.L.-Einsatz geprüft. Dazu wurden die Harzlösungen mit einem sauren Aminsalz (p-Toluolsulfonsäure/Morpholin) auf eine Trübungszeit von ca. 5 min eingestellt. Die mit diesen Harzlösungen imprägnierten Dekor- (PWA, A-60 S, 80 g/m², Harzauftrag ca. 60%, Restfeuchte ca. 6%) bzw. Kernlagenpapiere (Natron Kraft, 135 g/m², Harzauftrag ca. 55%, Restfeuchte ca. 6,7%) wurden im Trockenschrank getrocknet und anschließend zu Laminaten, bestehend aus 1 Dekorfilm-Lage und 3 Kernlagen, verpreßt (35 s, 175 °C, 3,5 MPa [=35 bar], Preßblech HS-18). Die Ergebnisse der Ausprüfung sind untenstehender Tabelle zu entnehmen:

| Harz | Ausprüfung der Laminate | | | | | |
|---|---|---|---|---|---|---|
| | Oberfläche | Kitontest 2 h | Dampftest * 60 min | Kochtest^{#} 2 h | Kochtest ^{#} 6 h | Post-Forming⁺ |
| Beispiel 7 | ohne Befund | 3 | ohne Befund | ohne Befund | ohne Befund | ohne Befund |
| Vergl. Bsp. 1 | ohne Befund | 2 | ohne Befund | ohne Befund | ohne Befund | ohne Befund |

| | | | | | | |
|---|---|---|---|---|---|---|
| * gemäß DIN-EN 438, Abschnitt 24 | | | | | | |
| # gemäß DIN-EN 438, Abschnitt 7 | | | | | | |
| + gemäß DIN-EN 438, Abschnitt 20 | | | | | | |

Zwischen dem erfindungsgemäßen Harz aus Beispiel 7 und dem Harz des Standes der Technik aus Vergleichsbeispiel 1 bestehen für den Einsatz zur Laminatherstellung keine anwendungstechnischen Unterschiede.

## Patentansprüche

1. N-Alkoxymethyl-Lactame der Formel VI 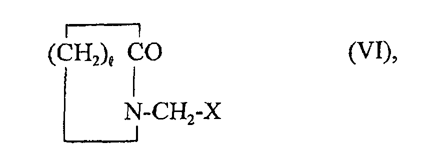 wobei X -(OCH₂-CR¹H)ₘ-OR² bedeutet und R¹ und R² jeweils unabhängig voneinander Wasserstoff oder einen linearen oder verzweigten Alkylrest mit 1 bis 6 Kohlenstoffatomen bedeuten, ℓ für 3 bis 12 und m für 1 bis 20 steht,
oder wobei X -O-(CH₂)ₙ-OR³ bedeutet, und R³ Wasserstoff oder einen linearen oder verzweigten Alkylrest mit 1 bis 6 Kohlenstoffatomen bedeutet, und n für 2 bis 8 steht.

2. Verfahren zur Herstellung von N-Alkoxymethyl-Lactamen der Formel VI des Anspruchs 1, **dadurch gekennzeichnet, daß**
Lactame der Formel I 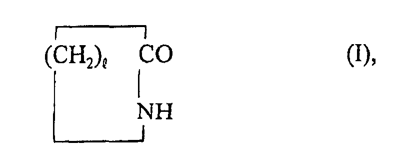 wobei ℓ für 3 bis 12 steht,
mit Formaldehyd und
Hydroxylgruppen-haltigen Verbindungen ausgewählt aus
- Diolen bzw. Diol-mono-äthern der Formel II
H(-O-CH₂-CR¹H)ₘ-OR² (II),
wobei R¹, R² jeweils unabhängig voneinander Wasserstoff oder einen linearen oder verzweigten Alkylrest mit 1 bis 6 Kohlenstoffatomen bedeuten, und m für 1 bis 20 steht, und
- Diolen bzw. Diol-mono-äthem der Formel III
HO-(CH₂)ₙ-OR³ (III),
wobei R³ Wasserstoff oder einen linearen oder verzweigten Alkylrest mit 1 bis 6 Kohlenstoffatomen bedeutet, und n für 2 bis 8 steht,
in Gegenwart von sauren Katalysatoren in einer einstufigen Reaktion bei Temperaturen von 50 bis 200 °C umgesetzt werden in einem Stoffmengenverhältnis von 1 mol des Lactams I zu 1 bis 4 mol Formaldehyd zu 0,5 bis 6 mol des Diols bzw. Diol-monoäthers II oder III.

3. Verfahren zur Herstellung von N-Alkoxymethyl-Lactamen der Formel VI des Anspruchs 1 in zwei Stufen, **dadurch gekennzeichnet, daß** in der ersten Stufe
Lactame der Formel I 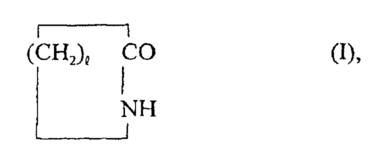 wobei *ℓ* für 3 bis 12 steht,
mit Formaldehyd in einem Stoffmengenverhältnis von 1 mol des Lactams I zu 1 bis 4 mol Formaldehyd in Gegenwart von alkalischen Katalysatoren bei Temperaturen von 50 bis 200 °C zu den entsprechenden N-Methylol-Lactamen umgesetzt werden, und
in der zweiten Stufe die N-Methylol-Lactame mit Hydroxylgruppen-haltigen Verbindungen ausgewählt aus
- Diolen bzw. Diol-mono-äthern der Formel II
H(-O-CH₂-CR¹H)ₘ-OR² (II),
wobei R¹, R² jeweils unabhängig voneinander Wasserstoff oder einen linearen oder verzweigten Alkylrest mit 1 bis 6 Kohlenstoffatomen bedeuten, und m für 1 bis 20 steht, und
- Diolen bzw. Diol-mono-äthern der Formel III
HO-(CH₂)ₙ-OR³ (III),
wobei R³ Wasserstoff oder einen linearen oder verzweigten Alkylrest mit 1 bis 6 Kohlenstoffatomen bedeutet, und n für 2 bis 8 steht,
in einem Stoffmengenverhältnis von 1 mol des Lactams I zu 0,5 bis 6 mol des Diols bzw. Diol-monoäthers II oder III in Gegenwart von sauren Katalysatoren bei Temperaturen von 50 bis 200 °C umgesetzt werden.

4. Verfahren nach einem der Ansprüche 2 oder 3, **dadurch gekennzeichnet, daß** das bei der Umsetzung gebildete Wasser während der Umsetzung oder nach der Umsetzung abdestilliert wird.

5. Gemische erhältlich durch das Verfahren nach Anspruch 2 oder 3, enthaltend N-Alkoxymethyl-Lactame der Formel VI 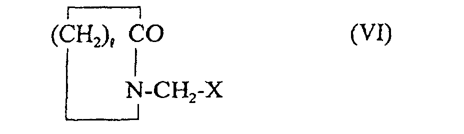 und N,N'-Methylen-Bis-Lactame der Formel V 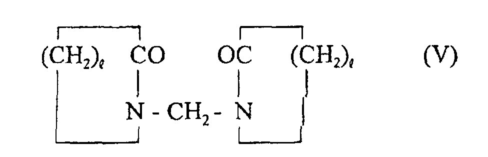 worin X für -(OCH₂-CR¹H)ₘ-OR² oder -O-(CH₂)ₙ-OR³ steht,
R¹, R² und R³ unabhängig voneinander Wasserstoff oder einen linearen oder verzweigten Alkylrest mit 1 bis 6 Kohlenstoffatomen bedeuten und ℓ für eine ganze Zahl von 3 bis 12, m für 1 bis 20 und n für 2 bis 8 steht.

6. Gemisch nach Anspruch 5, enthaltend Massenanteile von 5 bis 50 % N-Alkoxymethyl-Lactamen der Formel VI, 30 bis 80 % von N,N'-Methylen-bis-Lactamen der Formel V und 0 bis 5 % von nicht umgesetzten Lactamen der Formel I.

7. Verwendung der N-Alkoxymethyl-Lactame der Formel VI nach Anspruch 1 als Zusatz zu Melamin-Tränkharzen.

8. Verwendung von Gemischen nach Anspruch 5 als Zusatz zu Melamin-Tränkharzen.

9. Melamin-Tränkharz-Zubereitungen, enthaltend N-Alkoxymethyl-Lactame der Formel VI nach Anspruch 1 als Modifizierungsmittel in einem Massenverhältnis von 1 bis 20 %, bezogen auf die Masse des festen Melaminharzes.

10. Melamin-Tränkharz-Zubereitungen, enthaltend ein Gemisch nach Anspruch 5 als Modifizierungsmittel in einem Massenverhältnis von 1 bis zu 20 %, bezogen auf die Masse des festen Melaminharzes.

## Claims

1. An N-alkoxymethyllactam of the formula VI 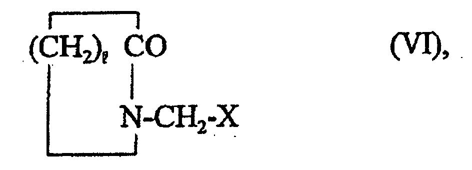 where X is -(OCH₂-CR¹H)ₘ-OR² and R¹ and R² in each case independently of one another are hydrogen or a linear or branched alkyl radical having 1 to 6 carbon atoms, ℓ is 3 to 12 and m is 1 to 20,
or where X is -O-(CH₂)ₙ-OR³ and R³ is hydrogen or a linear or branched alkyl radical having 1 to 6 carbon atoms and n is 2 to 8.

2. A process for preparing an N-alkoxymethyllactam of the formula VI of claim 1, which comprises reacting a lactam of the formula I 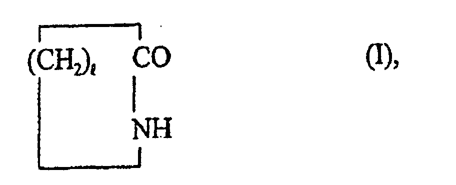 where ℓ is 3 to 12, with formaldehyde and hydroxyl-containing compounds selected from
- diols and/or diol monoethers of formula II
H(-O-CH₂-CR¹H)ₘ-OR² (II),
where R¹ and R² in each case independently of one another are hydrogen or a linear or branched alkyl radial having 1 to 6 carbon atoms, and m is 1 to 20, and
- diols and/or diol monoethers of formula III
HO-(CH₂)ₙ-OR³ (III)
where R³ is hydrogen or a linear or branched alkyl radical having 1 to 6 carbon atoms, and n is 2 to 8,
in the presence of acidic catalysts in a single-stage reaction at temperatures from 50 to 200°C in an amount-of-substance ratio of 1 mol of the lactam I to from 1 to 4 mol of formaldehyde to from 0.5 to 6 mol of the diol and/or diol monoether II or III.

3. A process for preparing an N-alkoxymethyllactam of the formula VI of claim 1 in two stages, which comprises in the first stage reacting a lactam of the formula I 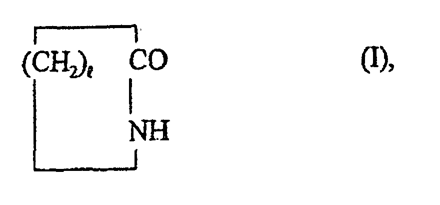 where ℓ is 3 to 12,
with formaldehyde in a amount-of-substance ratio of 1 mol of the lactam I or from 1 to 4 mol of formaldehyde in the presence of alkaline catalysts at temperatures from 50 to 200°C to give the corresponding N-methylollactam, and
in the second stage reacting the N-methylollactam with hydroxyl-containing compounds selected from
- diols and/or diol monoethers of formula II
H(-O-CH₂-CR¹H)ₘ-OR² (II),
where R¹ and R² in each case independently of one another are hydrogen or a linear or branched alkyl radial having 1 to 6 carbon atoms, and m is 1 to 20 and
- diols and/or diol monoethers of formula III
HO- (CH₂)ₙ-OR³ (III)
where R³ is hydrogen or a linear or branched alkyl radical having 1 to 6 carbon atoms, and n is 2 to 8,
in the presence of acidic catalysts at temperatures from 50 to 200°C in an amount-of-substance ratio of 1 mol of the lactam I to from 0.5 to 6 mol of the diol and/or diol monoether II or III.

4. The process as claimed in either of claims 2 and 3, wherein the water formed during the reaction is distilled off during the reaction or after the reaction.

5. A mixture obtainable by the process as claimed in claim 2 or 3, comprising N-alkoxymethyllactams of the formula VI 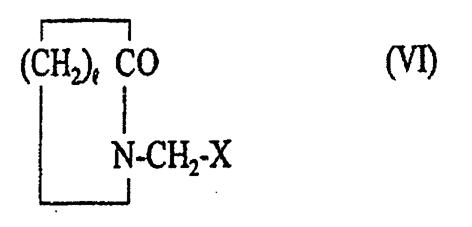 and N,N'-methylenebislactams of the formula V 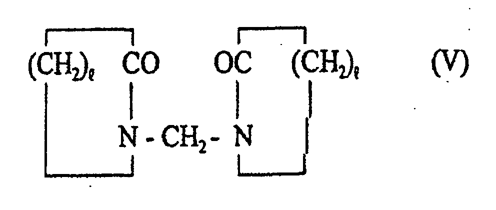 in which X is -(OCH₂-CR¹H)ₘ-OR² or -O-(CH₂)ₙ-OR³,
R¹, R² and R³ independently of one another are hydrogen or a linear or branched alkyl radical having 1 to 6 carbon atoms and ℓ is an integer from 3 to 12, m is 1 to 20 and n is 2 to 8.

6. The mixture as claimed in claim 5, comprising mass fractions of from 5 to 50% of N-alkoxymethyllactams of the formula VI, from 30 to 80% of N,N'-methylenebislactams of the formula V and from 0 to 5% of unreacted lactams of the formula I.

7. The use of an N-alkoxymethyllactam of the formula VI as claimed in claim 1 as an additive to melamine impregnating resins.

8. The use of a mixture as claimed in claim 5 as an additive to melamine impregnating resins.

9. A melamine impregnating resin formulation comprising an N-alkoxymethyllactam of the formula VI as claimed in claim 1 as modifier in a proportion by mass of from 1 to 20%, based on the mass of the solid melamine resin.

10. A melamine impregnating resin formulation comprising a mixture as claimed in claim 5 as modifier in a proportion by mass of from 1 to 20%, based on the mass of the solid melamine resin.

## Revendications

1. N-Alkoxyméthyl-lactames de formule VI 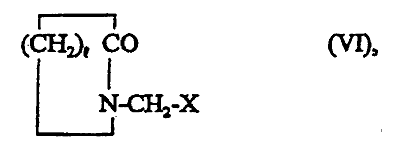 où X représente un groupe -(OCH₂-CR¹H)ₘ-OR² et R¹ et R² représentent chacun, indépendamment l'un de l'autre, un atome d'hydrogène ou un reste alkyle linéaire ou ramifié présentant 1 à 6 atomes de carbone, ℓ va de 3 à 12 et m va de 1 à 20,
où X représente un groupe -O-(CH₂)ₙ-OR³ et R³ représente un atome d'hydrogène ou un reste alkyle linéaire ou ramifié présentant 1 à 6 atomes de carbone et n va de 2 à 8.

2. Procédé pour la préparation de N-alkoxyméthyl-lactames de formule VI de la revendication 1, **caractérisé en ce qu'**on transforme des lactames de formule I 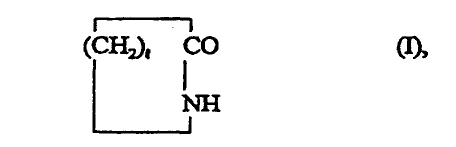 où ℓ va de 3 à 12,
par le formaldéhyde et
des composés présentant des groupes hydroxyle pris parmi
- des diols ou diol-monoéthers de formule II
H (-O-CH₂-CR¹H)ₘ-OR² (II)
où R¹, R² indépendamment, l'un de l'autre, représentent un atome d'hydrogène ou un reste alkyle linéaire ou ramifié présentant 1 à 6 atomes de carbone, et m va de 1 à 20, et
- des diols ou diol-monoéthers de formule III
HO-(CH₂)ₙ-OR³ (III)
où R³ représente un atome d'hydrogène ou un reste alkyle linéaire ou ramifié présentant 1 à 6 atomes de carbone et n va de 2 à 8,
en présence de catalyseurs acides dans une réaction en une étape à une température de 50 à 200°C, dans un rapport quantitatif de matières de 1 mole du lactame I à 1 à 4 moles de formaldéhyde à 0, 5 à 6 moles du diol ou diol-monoéther II ou III.

3. Procédé pour la préparation de N-alkoxyméthyl-lactames de formule VI de la revendication 1 en deux étapes, **caractérisé en ce qu'**on fait réagir dans la première étape des
lactames de formule I 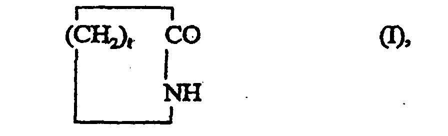 où ℓ va de 3 à 12,
sur le formaldéhyde dans un rapport quantitatif de matières de 1 mole du lactame à 1 à 4 moles de formaldéhyde en présence de catalyseurs alcalins à une température de 50 à 200°C pour obtenir les N-méthylollactames correspondants, et
dans la seconde étape, on fait réagir les N-méthylollactames sur des composés présentant des groupes hydroxyle pris parmi
- des diols ou diol-monoéthers de formule II
H(-O-CH₂-CR¹H)ₘ-OR² (II)
où R¹, R², indépendamment l'un de l'autre, représentent un atome d'hydrogène ou un reste alkyle linéaire ou ramifié présentant 1 à 6 atomes de carbone, et m va de 1 à 20, et
- des diols ou diol-monoéthers de formule III
HO- (CH₂)-OR³ (III)
où R³ représente un atome d'hydrogène ou un reste alkyle linéaire ou ramifié présentant 1 à 6 atomes de carbone et n va de 2 à 8,
dans un rapport quantitatif de matières de 1 mole du lactame I à 0, 5 à 6 moles du diol ou diol-monoéther II ou III en présence de catalyseurs acides à une température de 50 à 200°C.

4. Procédé selon l'une des revendications 2 ou 3, **caractérisé en ce qu'**on chasse par distillation l'eau formée au cours de la réaction ou après la réaction.

5. Mélanges que l'on peut obtenir à l'aide du procédé selon les revendications 2 ou 3, renfermant des N-alkoxyméthyl-lactames de formule (VI) 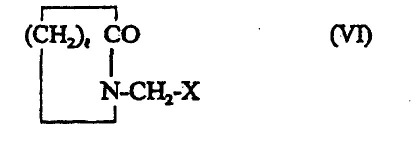 des N,N'-méthylène-bis-lactames de formule V 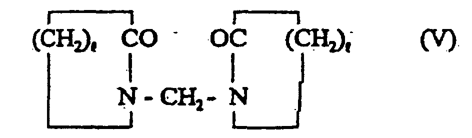 où X représente un groupe -(OCH₂-CR¹H)ₘ-OR² ou HO- (CH₂)ₙ-OR³,
R¹, R² et R³ indépendamment les uns des autres représentent un atome d'hydrogène ou un reste alkyle linéaire ou ramifié présentant 1 à 6 atomes de carbone et ℓ va de 3 à 12 et m va de 1 à 20 va de 2 à 8.

6. Mélange selon la revendication 5, renfermant des taux en masse de 5 à 50 % de N-alkoxyméthyl-lactames de formule VI, de 30 à 80 % de N,N'-méthylène-bis-lactames de formule V et de 0 à 5 % en masse de lactames n'ayant pas réagi de formule I.

7. Utilisation de N-alkoxyméthyl-lactames de formule VI selon la revendication 1 en tant qu'additifs de résines d'imprégnation de mélamine.

8. Utilisation de mélanges selon la revendication 5 en tant qu'additifs de résines d'imprégnation de mélamine.

9. Préparations de résines d'imprégnation de mélamine renfermant des N-alkoxyméthyl-lactames de formule VI selon la revendication 1 comme agent de modification dans un rapport en masse de 1 à 20 %, par rapport à la masse de la résine de mélamine solide.

10. Préparations de résines d'imprégnation de mélamine renfermant un mélange selon la revendication 5 comme agent de modification dans un rapport en masse de 1 à 20 %, par rapport à la masse de la résine de mélamine solide.
